(19) Europäisches Patentamt / European Patent Office / Office européen des brevets

(11) **EP 3 191 800 B1**

(12) **EUROPEAN PATENT SPECIFICATION**

(45) Date of publication and mention of the grant of the patent:
**18.08.2021 Bulletin 2021/33**

(21) Application number: **15774707.2**

(22) Date of filing: **27.08.2015**

(51) Int Cl.:
***G01D 5/353*** *(2006.01)*

(86) International application number:
**PCT/IB2015/056490**

(87) International publication number:
**WO 2016/038492 (17.03.2016 Gazette 2016/11)**

(54) **DETECTION OF SURFACE CONTACT WITH OPTICAL SHAPE SENSING**

DETEKTION EINES OBERFLÄCHENKONTAKTS MIT OPTISCHER FORMMESSUNG

DETECTION DE CONTACT DE SURFACE AVEC DETECTION DE FORME OPTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.09.2014 US 201462047363 P**

(43) Date of publication of application:
**19.07.2017 Bulletin 2017/29**

(73) Proprietor: **Koninklijke Philips N.V.
5656 AG Eindhoven (NL)**

(72) Inventors:
• **FLEXMAN, Molly Lara
  NL-5656 AE Eindhoven (NL)**
• **NOONAN, David Paul
  NL-5656 AE Eindhoven (NL)**
• **RAMACHANDRAN, Bharat
  NL-5656 AE Eindhoven (NL)**

(74) Representative: **Philips Intellectual Property &
Standards
High Tech Campus 5
5656 AE Eindhoven (NL)**

(56) References cited:
**WO-A1-2009/023801        WO-A1-2009/023801
WO-A1-2012/025856        WO-A1-2012/025856
WO-A2-2007/015139        WO-A2-2007/015139
US-A1- 2009 158 852       US-A1- 2009 158 852
US-B1- 8 622 935          US-B1- 8 622 935**

**Description**

**BACKGROUND:**

**Technical Field**

This disclosure relates to medical instruments and more particularly to shape sensing optical fibers employed in detecting interactions with surfaces.

**Description of the Related Art**

Optical shape sensing (OSS) uses light along a multicore optical fiber for device localization and navigation during surgical intervention. One principle involved makes use of distributed strain measurement in the optical fiber using characteristic Rayleigh backscatter or controlled grating patterns. The shape along the optical fiber begins at a specific point along the sensor, known as the launch or z = 0 and extends to the tip of the fiber. For clinical use, the shape sensing fiber is integrated into a medical device, such as a catheter, guide wire, endoscope, robotic tool, etc. This is typically done by placing the fiber into a lumen within the wall of the device.

Multiple shape parameters are provided from a reconstruction of the shape sensing data. These parameters include x, y, z position, axial strain, and twist, among others. Ultimately, all of these parameters are derived from measurements of the phase from multiple cores (for example, 4 cores) within a shape sensing fiber. The shape sensing measurement uses data from a range of illumination frequencies (for example, 20nm) that are swept using an input light source. This needs some finite amount of time (for example, 1-10ms) to perform a measurement. During that time, changes in the phase in the cores can lead to an incorrect reading and corresponding incorrect shape reconstruction. During navigation, these incorrect shapes are typically detected and removed. Since the twist is an average of the three outer cores normalized by the central core, it provides an aggregate of the phase in all of the cores. It is also know to retrieve force components from optical sensing data (US8622935 and WO2007/015139).

In endovascular procedures, there is a risk of producing embolic particles due to the scraping of the interventional devices during navigation. These embolic particles that are dislodged during navigation may lead to adverse conditions, such as clots in other regions of the vasculature. Clots may in turn lead to adverse events such as stroke. It is difficult for operators to know how much contact the tip of the device is making with the vessel wall during navigation.

**SUMMARY**

The invention is solely defined by the appended claims. Further examples that are not encompassed in the scope of the claims, even if they are indicated as "embodiments of the invention" in the description, are not part of the invention. In accordance with the present principles, a system for detecting instrument interaction with a surface includes a shape sensing enabled instrument configured to pass along the surface. An interaction evaluation module is configured to monitor shape sensing feedback from the instrument to determine modes of the shape sensing feedback that identify whether contact is made with the surface and to determine the position of the contact.

A system for detecting instrument interaction with a surface includes a flexible instrument configured to pass along a surface. An optical shape sensing system is integrated into the instrument and configured to provide an optical shape sensing signal as shape sensing feedback. A processor and memory coupled to the processor are included. An interaction evaluation module is stored in the memory and configured to monitor the shape sensing feedback from the instrument to determine modes of the shape sensing feedback that identify whether contact is made with the surface. An actuation module is configured to adjust the instrument in accordance with the modes of the shape sensing feedback.

A method for detecting instrument interaction with a surface includes receiving shape sensing feedback from a flexible shape sensing enabled instrument configured to pass along the surface; and evaluating the shape sensing feedback using an interaction evaluation module configured to monitor the shape sensing feedback from the instrument to determine modes of the shape sensing feedback in order to identify whether contact is made with the surface and to determine the position of the contact.

These and other objects, features and advantages of the present disclosure will become apparent from the following detailed description of illustrative embodiments thereof, which is to be read in connection with the accompanying drawings.

**BRIEF DESCRIPTION OF DRAWINGS**

This disclosure will present in detail the following description of preferred embodiments with reference to the following figures wherein:

FIG. 1 is a block/flow diagram showing a shape sensing system which employs an interaction evaluation module for detecting whether contact has been made by an instrument with a wall of a vessel in accordance with one embodiment;

FIG. 2 shows two examples of instrument-to-vessel wall interactions;

FIG. 3 shows two twist profile plots showing a normal twist profile and an erroneous twist profile due to vibrational disturbance;

FIG. 4 shows a twist profile plot showing a distal tip region of the signal corrupted by vibrational disturbance;

FIG. 5 shows a tip vibration metric (tvm) plotted against frame number for three datasets where Dataset 1 shows no tip scraping inside a phantom, Dataset 2 shows wall scraping inside the phantom and Dataset 3 shows tip scraping against skin in accordance with the present principles; and

FIG. 6 is a block/flow diagram showing a method for detecting whether contact has been made by an instrument with a wall or surface in accordance with illustrative embodiments.

## DETAILED DESCRIPTION OF EMBODIMENTS

In accordance with the present principles, systems and methods are disclosed to identify when a part of the interventional device has made contact with a surface, such as, a vessel wall, a surface of the skin, a heart, a bone, a non-biological surface, etc. Such techniques may preferably employ an optical shape sensing signal, but multiple approaches may be employed. In some embodiments, a combination of techniques including but not limited to optical shape sensing may be employed to provide a meaningful representation of surface contact or wall scraping. Wall scraping information can be extracted from the optical shape sensing signals in a plurality of ways. These may include, e.g., identifying the presence of vibrations through discontinuities in a twist signal, identifying the presence of vibrations through frequency components of the twist signal, detecting compression at the tip of the device from an axial strain signal, predicting wall contact from the shape of a device - specifically high curvature in a distal section, using a motion profile of the device to isolate signals indicating contact, etc.

Navigation of medical devices into target vessels may be achieved using pre-curved catheters and guidewires, which interact with each other and the vascular wall. This interaction with the vascular wall can lead to the creation of embolic particles that are dislodged during navigation. Such particles can result in clots in other regions of the vasculature, leading to adverse events such as stroke. In conventional scenarios, it is difficult for operators to know how much contact the tip of the device is making with the vessel wall during navigation since the devices are manipulated from outside the body and any highfrequency vibrations induced by the tip contact are dampened out before being sensed by the operator. Further, in robotic procedures where the operator no longer has tactile feedback from the device, it is especially important to know the interactions between the tip of the device and the vessel wall.

Vibrations occurring during an optical shape sensing measurement are acquired within the shape sensing parameters. For example, a twist parameter tends to show discontinuities and spikes in the presence of vibration. These vibrations can be spatially localized along the length of the sensor. Similarly, vibration can manifest itself as a broadening of the termination reflection measured in the fiber. Another shape parameter, curvature, can indicate contact with the wall when the radius of curvature becomes very small in the distal portion of the device. By inspecting the shape sensing data for manifestation of these and other features in accordance with the present principles, it is possible to quantify the amount of wall scraping occurring during navigation. In the case of manual operation, this value could be reported to the operator.

For robotic operation, this information could be part of the control loop to encourage an alternative position or shape for the distal part of the device. For example, an actuation module may be employed that employs information about a shape sensing enabled instrument to determine, e.g., when the instrument is making contact with the vessel wall and reduce or prevent such contact, to predict when the contact is going to be made and lower its impact (force), to determine whether sufficient contact is being made with a surface, such as the surface of a bone or skin to paint a feature for an orthopedic application like total knee replacement or other procedure (for example, when scraping tissue, the present principles can determine if bone has been contacted versus skin or muscle, etc.).

It should be understood that the present invention will be described in terms of medical instruments; however, the teachings of the present invention are much broader and are applicable to any fiber optic instruments. In some embodiments, the present principles are employed in tracking or analyzing complex biological or mechanical systems. In particular, the present principles are applicable to internal tracking procedures of biological systems, procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

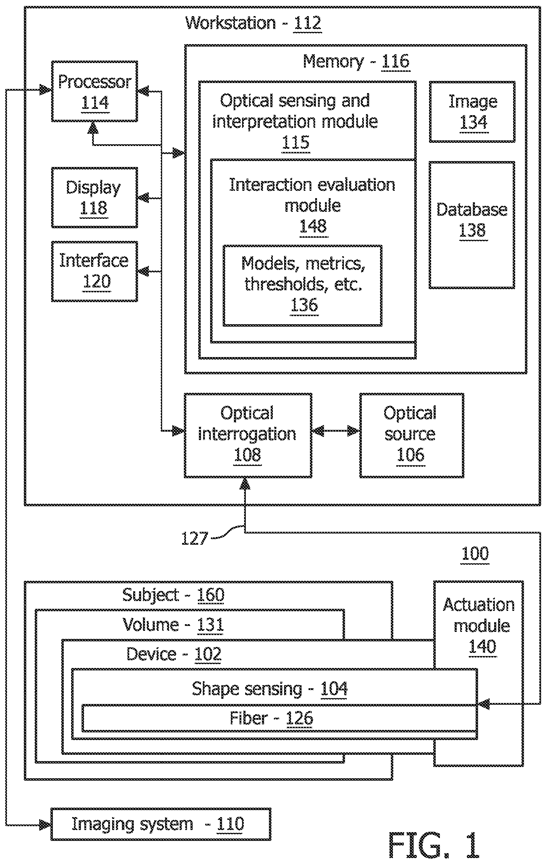

Referring now to the drawings in which like numerals represent the same or similar elements and initially to FIG. 1, a system 100 for detecting surface contact using shape sensing enabled instruments is illustratively shown in accordance with one embodiment. System 100 may include a workstation or console 112 from which a procedure is supervised and/or managed. Workstation 112 preferably includes one or more processors 114 and memory 116 for storing programs and applications. Memory 116 may store an optical sensing module 115 configured to interpret optical feedback signals from a shape sensing device or system 104. Optical sensing module 115 is configured to use the optical signal feedback (and any other feedback, e.g., electromagnetic (EM) tracking) to reconstruct deformations, deflections and other changes associated with a medical device or instrument (shape sensing enabled instrument) 102 and/or its surrounding region. The medical instrument 102 may include a catheter, a guidewire, a probe, an endoscope, a robot, an electrode, a filter device, a balloon device, a pointer, or other medical component, etc.

The shape sensing system 104 on instrument 102 may include one or more optical fibers 126 which are coupled to the instrument 102 in a set pattern or patterns. The optical fibers 126 connect to the workstation 112 through cabling 127. The cabling 127 may include fiber optics, electrical connections, other instrumentation, etc., as needed. The cabling 127 interfaces with an optical interrogation unit 108 that may include or work with an optical source or sources 106. The interrogation unit 108 sends and receives optical signals from the shape sensing system 104.

Shape sensing system 104 with fiber optics may be based on fiber optic Bragg grating sensors. A fiber optic Bragg grating (FBG) is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector.

Inherent backscatter in conventional optical fiber can be exploited for optical shape sensing (OSS). One such approach uses Rayleigh scatter (or other scattering) in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multi-core fiber, the 3D shape and dynamics of the surface of interest can be followed.

Fiber Bragg Gratings (FBGs) may also be employed for OSS, which use Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optic sensors. In an FBG sensor, the measurand (e.g., strain) causes a shift in the Bragg wavelength.

One advantage of OSS is that various sensor elements can be distributed over the length of a fiber. Incorporating three or more cores with various sensors (gauges) along the length of a fiber that is embedded in a structure permits a three-dimensional form of such a structure to be precisely determined with high accuracy. Along the length of the fiber, at various positions, a multitude of FBG sensors can be located (e.g., 3 or more fiber sensing cores). From the strain measurement of each FBG, the curvature of the structure can be inferred at that position. From the multitude of measured positions, the total three-dimensional form is determined.

In one embodiment, the optical sensing module 115 includes an interaction evaluation module 148. Data collected from the OSS device 104 is interpreted to evaluate an amount of wall interaction/contact made by the instrument 102 using the OSS device 104 feedback. The wall interaction evaluation module 148 may focus on one or more different parameters to measure the duration, force, severity, or magnitude of the interaction, etc. made by the instrument 102 and a vessel or organ wall in a volume 131 where the instrument 102 is deployed. The surface interaction evaluation module 148 is configured to receive feedback from the shape sensing device 104 and record position and orientation data as to where the sensing device 104 has been within the volume 131. Position (and orientation) data or other data from the shape sensing device 104 within the space or volume 131 can be displayed on a display device 118. Workstation 112 includes the display 118 for viewing internal images 134 of a subject (patient) 160 or volume 131 and may include the image 134 as an overlay or other rendering of the positions of the shape sensing device 104 on images collected by an imaging device 110. The imaging device 110 may include any imaging system. Display 118 may also permit a user to interact with the workstation 112 and its components and functions, or any other element within the system 100. This is further facilitated by an interface 120 which may include a keyboard, a mouse, a joystick, a haptic device, or any other peripheral or control to permit user feedback from and interaction with the workstation 112.

The wall interaction evaluation module 148 evaluates clinically useful data to determine when, where and how much surface interaction (e.g., wall scraping) takes place during a procedure. In particularly useful embodiments, techniques employed to identify when a part of the interventional instrument 102 has made contact with a surface (e.g., a vessel wall, skin, bone, organ or vessel wall scraping) can be extracted from the optical shape sensing signals of the OSS system 104. The techniques may include identifying the presence of vibrations through discontinuities in a twist signal, identifying the presence of vibrations through frequency components of the twist signal, and/or detecting compression at the tip of the instrument 102 from the axial strain signal. Other techniques include predicting wall contact from the shape of the device. This may include determining high curvature in the distal section of the instrument 102 or other shapes depending on the physical constraints and conditions. In other embodiments, the motion profile of the instrument 102 may be employed to understand when wall interaction has occurred.

The wall interaction evaluation module 148 includes models, algorithms, formulas, etc. that look at specific data to understand when portions of the instrument 102 engage the walls or surfaces of the volume 131 using the OSS signal of the OSS device or system 104. When the wall interaction evaluation module 148 determines that engagement has occurred, the severity and duration of the engagement may be evaluated using the data. The data may be compared against acceptable thresholds and be employed to provide guidance to the user during a procedure. For example, if too much wall interaction is measured, there may be an obstruction or other issue and the procedure may be terminated or the instrument 102 withdrawn. In other embodiments, other actions may be taken preferably in accordance with the guidance provided from the interaction evaluation module 148 to the user.

In one embodiment, where computer-aided or robotic assistance is employed, information employed by the interaction evaluation module 148 could be used as part of the control loop to encourage an alternative position or shape for a part of the instrument 102 (e.g., the distal end portion). For example, an actuation module 140 may be employed to control motion or shape of the instrument 102. The actuation module 140 employs information provided by the shape sensing enabled instrument 102 to determine, e.g., when the instrument 102 is making contact with a surface or vessel wall, and to reduce or prevent such contact (e.g., change the shape of the instrument 102). The actuation module 140 may provide feedback (e.g., vibration, light, audible alarm, etc.) to a user that surface contact has been made or that surface contact exceeding a threshold (e.g., force threshold) has been made. In another embodiment, the actuation module 140 may be employed to alter the position or use of the instruments due to a prediction as to when contact is going to be made (by the interaction evaluation module 148). Predictions may be made using prior data in models 136, etc. The actuation module 140 would than attempt to lower its impact (force) by changing direction, reducing speed, etc. The actuation module 140 may be employed as a sensor to determine whether sufficient or significant contact is being made with a surface, such as the surface of a bone or skin, e.g., to paint a feature for an orthopedic application like total knee replacement or other procedure.

The actuation module 140 may include hardware systems configured to advance or retract the instrument at defined displacements, velocities and/or accelerations. The actuation module 140 may include robotic control mechanisms, such as actuators, servos, pneumatics, etc.

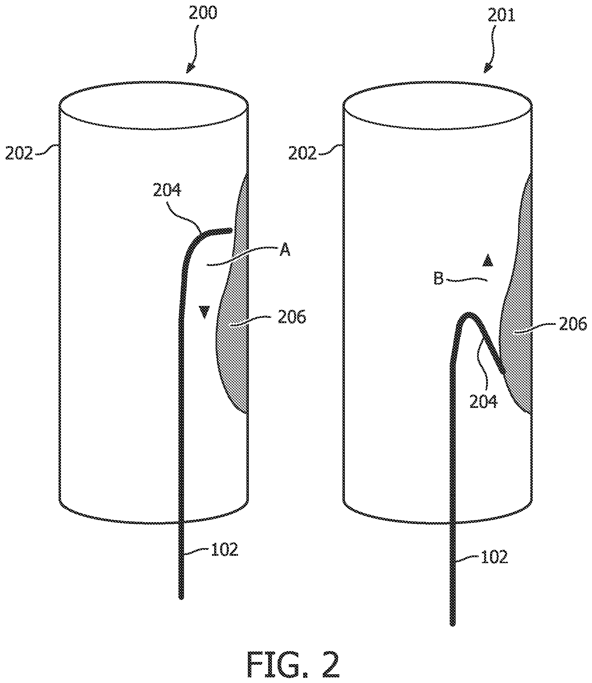

Referring to FIG. 2, examples 200 and 201 of a shape sensing enabled instrument 102 in a blood vessel 202 are depicted to demonstrate tip interaction with internal surfaces of the blood vessel 202. In example 200, the instrument 102 is pulled back along the vessel 202 in the direction of arrow "A". A tip 204 scrapes against a plaque 206. To prevent embolic particles from being dislodged into the blood stream, the present principles are employed to determine whether surface contact is being made. In example 201, the instrument 102 is advanced up the vessel 202 in the direction of arrow "B". The tip 204 is bent back by the contact with the vessel wall 202. This also causes interaction with the plaque, and the potential dislodgement of particles into the blood stream. In both examples 200 and 201, the shape and configuration of the device 102 may be employed to determine the surface interaction. Surface interaction can be determined by identifying the presence of vibrations through discontinuities in the twist signal or through frequency components of the twist signal, curvature detection, motion profile and/or detecting compression at the tip 204 of the instrument 102 from the axial strain signal. Other techniques may also be employed, e.g., wall contact prediction from the shape of the instrument (e.g., high curvature in the distal section, etc.).

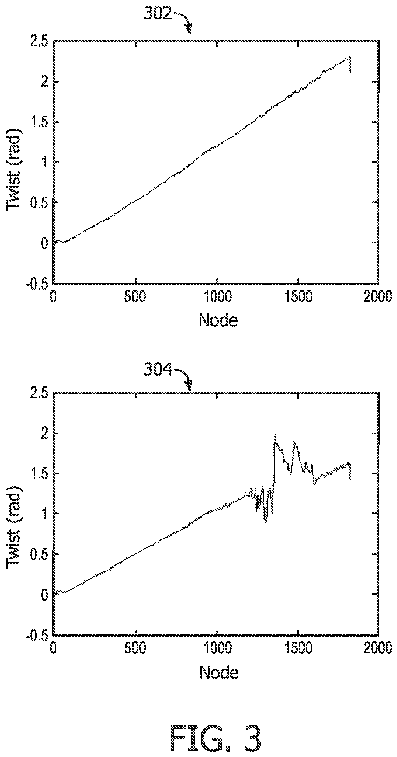

Referring to FIG. 3, a twist profile provides an amount of twist in the OSS system 104 (and therefore the instrument 102). FIG. 3 shows twist in radians plotted against node number where node number increases distally (to the right in the profile). When the tip of the instrument 102 comes in contact with a wall or surface and is scraped along it, there can be a vibration imparted onto the tip of the instrument 102. This vibration is typically isolated to the distal portion of the OSS shape sensor and results in an incorrect measurement in that region. Plot 302 shows an example of a normal twist profile, and plot 304 shows an erroneous twist profile that has been corrupted by vibration during the measurement. Shapes with a twist profile may be considered incorrect (an outlier) and are usually removed from the display (outlier rejection) in conventional systems. In many cases, a very distal portion of the sensor (e.g., 10-100 nodes or 0.8-8mm) is omitted from this type of outlier detection since it is typically sensitive to corruption by vibrations and can lead to an excessive rejection of clinically-useful data, especially since it does not contribute to the majority of the shape. In accordance with the present principles, the data collected for this very distal portion of the shape measurement can be employed to detect and measure vibrations occurring at the tip of an interventional instrument (102).

The tip vibrations can be caused by a mechanical interaction between the instrument and the vessel wall or surface. In robotic procedures, the operator often does not have tactile feedback from the instrument 102. In this case, it would be useful to know the interactions between the tip of the instrument 102 and the vessel wall. This could then be fed back to the operator as a 'vibration' felt in a robotic controller (e.g., actuation module 140). A robotic control loop can consider the amount of tip vibration in the positioning and distal shape of the instrument.

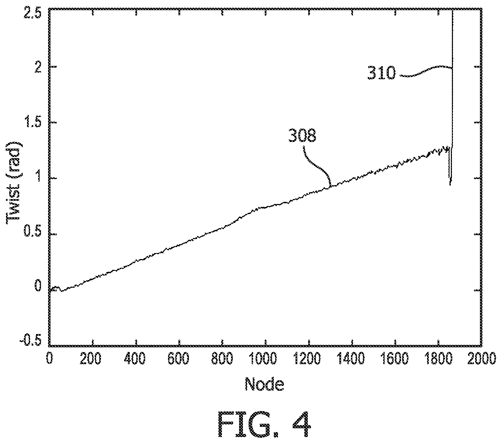

Referring to FIG. 4, a twist profile or curve 308 shows twist in radians plotted against node number where node number increases distally. Curve 308 shows a shape corrupted by vibration at the distal tip region during the measurement where the tip experiences tip vibration through wall contact during navigation. Note that there are observable discontinuities 310 in the distal nodes of the sensor.

To detect that the tip is experiencing vibration, a number of algorithms may be employed. For example, a threshold on the differential of the distal part of the twist, as shown below, may be employed:

$$tvm = \max(\theta_i - \theta_{i-10}) \quad \textit{for i=end-10....end} \quad\quad (1)$$

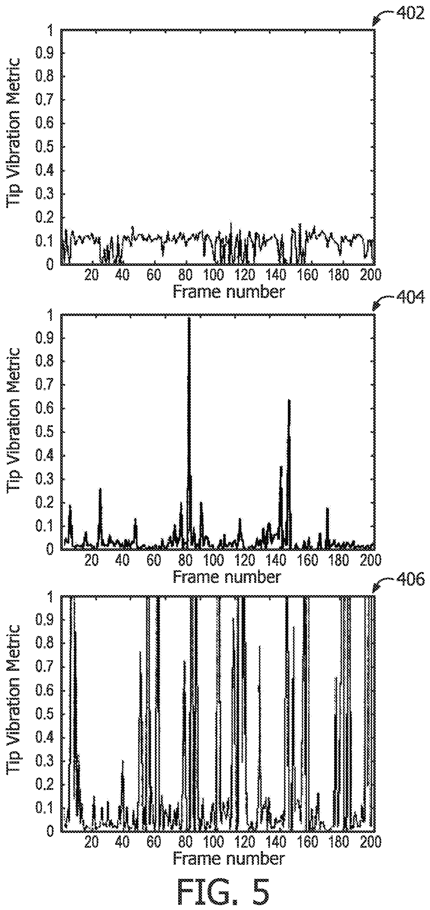

The tip vibration metric (*tvm*) is computed using Equation (1), where $\theta$ is the twist in radians, i is the node along the fiber and *end* is the number of nodes in the fiber (1858 in the example of FIG. 4). The algorithm tests the last 10 nodes of the fiber and uses the maximum jump in twist to identify a potential vibration. Twist is derived from the average phase difference of outer fiber cores normalized by a central fiber core of the OSS system, and therefore any algorithms described for the twist metric can also be applied to the phase difference from one or more cores. Referring to FIG. 5, the algorithm of Equation (1) was applied to 3 datasets. The datasets included:

Dataset 1: Navigation with a catheter in a vascular phantom (hard plastic used as a simulation) with no contact between the tip and the vessel wall. The tip of the catheter is free floating in the phantom vessel during navigation.
Dataset 2: Navigation with a catheter in the vascular phantom (e.g., hard plastic) with contact between the tip and the vessel wall. The tip of the catheter makes contact with the phantom wall during navigation.
Dataset 3: Manually dragging the tip of the catheter along a skin surface (palm of a hand).

A tip vibration metric (tvm) was computed for the three datasets and was respectively plotted in plots 402, 404 and 406. Shapes that had twist error in the proximal part of the shape were excluded from this analysis to only focus on the vibrations in the tip region. In the dataset with no tip scraping inside the phantom (Dataset 1), the tvm of plot 402 is very low during navigation. In the dataset with wall scraping inside the phantom (Dataset 2), there are clearly a greater number of large jumps in the tip vibration in plot 404. The interior of the phantom in this simulation was very smooth, hard plastic. In plot 406, the tip of the device was pulled across a skin surface, and there is a significant increase in the tvm. The percentage of shapes that exceeded a tvm threshold during a specific time interval (2 seconds), for example, could be reported to the operator for feedback on the amount of vibration experienced at the tip of the device. Other criteria and thresholds may also be employed.

Referring again to FIG. 4, twist frequency components may be employed as another way of detecting surface contact between the instrument 102 and the wall of a lumen. A method of detecting vibration at the tip / distal section of the instrument 102 and, in turn, wall scraping, is by making use of dampening along the length of the instrument 102.

When the tip of the device comes in contact with the vessel wall, a high frequency vibration indicated by discontinuities 310 is observed at the tip. This vibration can propagate, as a longitudinal wave, along the length of the device, while getting dampened as it travels further. The amplitude and frequency of the vibration, as well as the rate of dampening, vary and can be written as functions of the amplitude of the contact with the vessel wall, the duration of the scraping along the wall, the properties of the device such as its weight, structure, mechanical and material properties and so on. Once characterized, the amount of dampening (or other properties) can be employed to quantify the amount, duration and nature of the wall instrument interaction. In this example, the twist plot of FIG. 4, during wall scraping, shows a higher frequency component at the distal tip, with this frequency reduction showing dampening of vibration along the length of the device.

Another related way of picking up vibration is by evaluating the frequency of the twist signal near the tip of the instrument 102. The frequency of the distal section will be greater than the proximal portion. The frequency is expected to decrease along the length (from the tip towards the proximal region), and, using this scheme, vibration due to wall scraping can be distinguished from changes in twist due to other reasons such as problems with the termination and torqueing of the device. The distinguished signal can be employed to quantify the amount, duration and nature of the surface-instrument interaction.

In accordance with the present principles, other measurements may be made and employed to evaluate the occurrence, magnitude and duration on surface or wall interaction with an instrument. In one embodiment, axial tension may be measured and employed. In such a method for determining wall scraping, an axial tension signal is obtained during shape reconstruction. If the tip of the instrument 102 touches the wall, the interaction can result in a small compression at the tip. This contact can be determined from the optical sensing signal. If the tip touches the wall at an angle, a component of the force that is in an axial direction (along the direction of the fiber/instrument) will be observed in an axial tension signal and this could be used to determine wall scraping. In another embodiment, temperature can be used to identify contact with a warm or cold surface. Temperature can be extracted from the optical shape sensing parameters through the central core of the fiber. A temperature indicator is particularly relevant for applications in which a device is used outside the body to make contact with the skin surface.

In another embodiment, specific curvatures of the instrument 102 may be used to identify surface contact. Tip wall scraping can induce a small curvature in the distal portion of the sensor (104) and can indicate that the device is in contact with the vessel wall, as shown, e.g., in FIG. 2. This is primarily relevant for manual non-actuated devices where a small radius of curvature of the instrument 102 can only be the result of external mechanical interactions with the anatomy, etc.

In yet another embodiment, a motion profile may be employed to identify wall/instrument interaction. Wall scraping may be detected by observing the displacement, velocity and acceleration patterns (motion profile) of the OSS signal. Scraping of the tip of the instrument 102 is likely to cause sudden accelerations/decelerations of the distal portion of the instrument 102. This method may employ algorithms used to distinguish accelerations/decelerations due to normal handling with accelerations/decelerations due to wall scraping. This can be done by comparing acceleration/deceleration patterns at the tip (or portion in contact with the vessel wall) against other portions of the fiber that are not in contact with the vessel wall. Characterization data may be stored for comparison for use by the interaction evaluation module 148 (FIG. 1). When the motion profile is measured, it can be compared with the characterization data to determine whether an interaction has occurred and the type of interaction.

It should be understood that the description has been focused on the tip of the instrument 102; however, all of the methods/techniques described are applicable to more proximal parts of the instrument 102. It may be of interest to only identify vibrations that occur within the body as wall scraping, so knowing how much of the device is within the body is relevant.

Referring again to FIG. 1, the interaction evaluation module 148 may include models and/or composite metric modules 136 employed to interpret and combine measured results received from the shape sensing system 104. In one embodiment, a composite metric may be employed, which includes a combination of vibration and curvature detection measurements. These measurements may be combined in different ways including weighting the contributions from each to achieve a more accurate interaction determination and characterization. For example, each method employed for characterizing wall/instrument interactions may be given a score and the score may be weighted based on importance so that an overall score is derived. The overall score can be employed to compare against thresholds to identify the interaction. Based on the overall score or one or more individual scores, the interaction evaluation module 148 may be employed to make recommendations as the type and severity of the wall scraping or interaction. In one embodiment, recommendations may be made on how to minimize or reduce the wall scraping or interactions. For example, if an axial stress is sensed at the distal tip, the interaction evaluation module 148 may make a recommendation to straighten the instrument 102 to reduce this type of interaction. These actions may be performed by the actuation module 140.

The recommendations may be made based upon an indexed data storage system or relational database 138. When an overall score or individual score is computed, the score can be referenced in the database 138 to determine a recommended action. The recommended action may include pre-stored text, which can be displayed on the display 118, the data may be processed using a model or formula to output a specific action (e.g., "twist 30 degrees clockwise", etc.) or actions may be carried out by the actuation module 140. The score and recommendation can be based on the type of procedure, the known position of the device within the body using optical shape sensing and/or other imaging information (e.g., x-ray, CT, MRI, endoscopy, etc.).

In the case where multiple shape sensed devices are used together, known positions of the instruments (102) with respect to each other can be employed by the interaction evaluation module 148 to identify the more distally extended instrument that is more likely to be making contact with the vessel wall. Changes in the stiffness of the vessel wall will result in different 'vibration signatures' as the tip scrapes over the surface. Mechanical properties can be inferred by changes in the vibration of the tip as it passes from stiff tissue to a soft tissue, for example. In this case, the stiff tissue could be caused by stenosis and tip scraping would result in higher frequency vibrations than when scraping over the softer, healthy tissue region. Thus, the location of stenotic regions could be identified and indicated to the operator in real time. Such vibration patterns can be specific to the instrument 102 (different for guide wires and catheters) and this difference can be further used to identify the properties/texture/tissue-type of the region.

In addition, such stenotic regions could be matched to known stenotic regions identified from pre- or intra-operative imaging. This could be used to improve the registration between the shape sensing instrument and model being used for navigation purposes. The detection of tip scraping data to images and navigation models may be employed to provide other benefits as well. For example, positions of detected wall scrapings (from OSS fibers) may be employed to register vessel walls in stored or live images. In another example, the images may be employed to verify OSS data indicating wall contact.

In other embodiments, the present principles may be employed to turn the distal portion of OSS system 104 into a tip sensor. For example, the change in vibration signature of the distal portion may be employed to identify the position of a stent within a vessel either prior to full deployment or after deployment. In this embodiment, the vibration signature from the stent graft material would provide a different signature to the vascular wall on either side. Similarly, the location of a fenestration within a stent graft would be detectable due an abrupt change in vibration signature.

Imaging information from the imaging system 110, such as an X-ray image or a video image, may be employed to identify when the device tip touches the wall of a vessel. The imaging information may be mapped with the OSS information (e.g., twist, frequency of twist, dampening, axial strain, etc.), and a pattern or event (per vessel/device/individual) can be recorded using the combined data (image and OSS). The combined data may be employed to build a database. By building a database or using this information *a priori*, patterns of when tip wall scraping occurs can be obtained for given portions of a procedure. These patterns may be employed and stored in the database 138 to provide predictive tools for determining a likely portion of a procedure when wall scraping may occur. The database 138 can provide warning messages corresponding with the points in the procedure where greater care should be taken. The combined data (image and OSS) can also be applied as a search criterion to determine the instances of tip wall scraping.

In other embodiments, another way to determine wall scraping is by using dampened vibration. In such a case, a certain input vibration may be applied to the instrument 102 during navigation. This may be of a known pattern, amplitude, or frequency. This vibration would be transmitted (with some dampening) to the distal tip of the instrument 102, and any change in this pattern (point dampening for instance) would mean that the tip of the device has come in contact with the vessel wall. The input vibration may be the natural vibration of a robotic actuator that is manipulating the elongated device, such as, a catheter. In a non-robotic case, the vibration may be imparted by the clinician during normal handling and navigation. In both of these cases, the frequency of the vibrations, as well as the differential of the twist signal, among others, could be employed to predict the position and time of contact of the device with the vessel wall.

The present principles apply to a wide variety of applications including any integration of optical shape sensing technology into medical devices or other instruments for navigation in the body (e.g., endoscopes, bronchoscopes, catheters, guide wires, etc.) or through mechanical systems. This includes robotic and non-robotic use cases. In addition, the present principles find utility in non-medical applications, e.g., identifying when an instrument is making contact with a wall or a surface. The present principles apply to OSS systems that employ any scatter or reflective phenomena, e.g., Rayleigh (enhanced and regular) as well as Fiber Bragg implementations of shape sensing fiber.

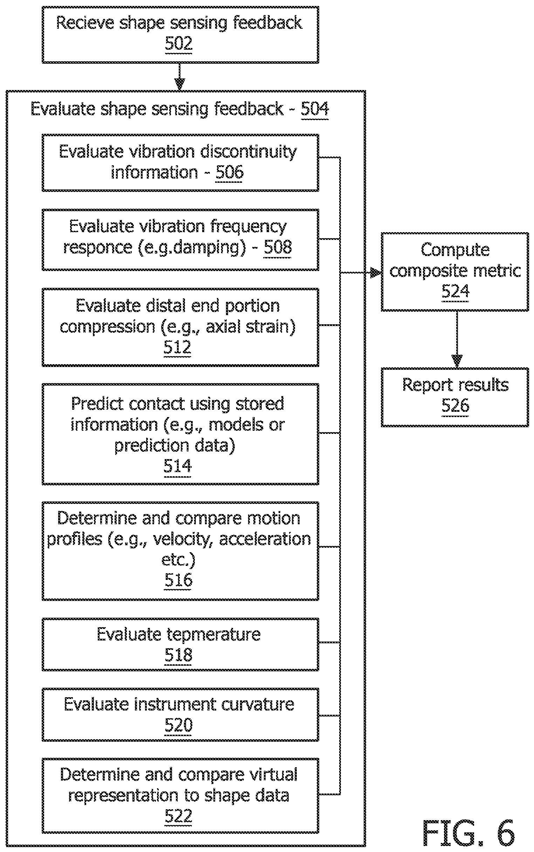

Referring to FIG. 6, a method for detecting instrument interaction with a vessel wall is shown and described. In block 502, shape sensing feedback is received from a flexible shape sensing enabled instrument configured to pass along a surface. The vessel may include any lumen, organ, surface, wall, skin, bone, muscle or mechanical component or volume. In block 504, the shape sensing feedback is evaluated using an interaction evaluation module configured to monitor the shape sensing feedback from the instrument to determine modes of the shape sensing feedback that identify whether contact is made with a surface or wall of a vessel. The evaluation may include one or more different parameters as will be illustratively described.

Evaluating the shape sensing feedback may include evaluating vibrations identified by discontinuity information in the optical shape sensing signal in block 506, or evaluating vibrations identified by frequency response information (e.g., damping response changes, etc.) in the optical shape sensing signal in block 508. Evaluating the shape sensing feedback may include identifying compression (e.g., axial strain) in the optical shape sensing signal at a distal end portion of the instrument in block 512, predicting contact between the instrument and the surface based on stored information (e.g., models, metrics, prediction data, etc.) in block 514 or determining and/or comparing a motion profile or images with stored data to determine whether contact is made between the instrument and the surface in block 516. OSS data may be compared to images, or images may be compared to OSS data to provide registration between the coordinate systems, or to verify contact with a surface, etc. In block 518, evaluating the shape sensing feedback may include identifying temperature differences (e.g., axial strain) in the optical shape sensing signal at a distal end portion of the instrument. In block 520, evaluating the shape sensing feedback may include identifying known curvatures in the optical shape sensing instrument.

In block 522, a representation (e.g., an image (stored or live), model, etc.) of the surface may be configured for comparison with positions and orientations determined by the optical shape sensing of the instrument. The comparison may be to determine whether surface contact has occurred or to identify a position where surface contact has occurred between the instrument and the surface. The images and the OSS data may be registered to determine if contact has occurred, or contact may be verified by registering the contact position with a boundary or vessel wall. In another embodiment, the OSS contact may be employed as a criterion to register the vessel wall or boundary. Block 522 may be performed independently for registration, navigation, data collection, etc. of the instrument relative to the surface, as needed.

In block 524, a composite metric may optionally be computed and is configured to determine interaction between the instrument and the surface using two or more parameters of the shape sensing feedback, e.g., two or more of the parameters of blocks 506-522. Other parameters may also be employed instead of or in addition to those included in blocks 506-522. For example, the two or more parameters of the composite metric may include a combination of vibrational information of the instrument, axial strain of the instrument, curvature of the instrument, temperature, motion patterns of the instrument, etc.

In block 526, results are reported. A type and magnitude of interaction between the instrument and the wall may be indicated to a user. This may include directions, actions or other information on how to avoid or reduce such contact, or simply provide real-time feedback in which the user can discontinue a present task or action to reduce wall scraping or the like.

In interpreting the appended claims, it should be understood that:

a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for detection of surface contact with optical shape sensing (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims.

## Claims

1. A system for detecting instrument interaction with a surface, comprising:

a shape sensing enabled instrument (102) configured to pass along the surface and which includes an optical shape sensing system (104) configured to provide an optical shape sensing signal as the shape sensing feedback; and
an interaction evaluation module (148) configured to monitor shape sensing feedback from the instrument to determine modes of the shape sensing feedback in order to identify whether contact is made with the surface and to determine the position of the contact, **characterized in that** the modes of shape sensing feedback include one or more of:

vibrations identified by one or more of **discontinuity** information or **frequency** response information in the optical shape sensing signal;
a **shape configuration** determined from the optical shape sensing signal indicating **contact** between the instrument (102) and the surface; and

a **motion profile** determined from the optical shape sensing signal indicating **contact** between the instrument (102) and the surface.

2. The system as claimed in claim 1, wherein the modes of the shape sensing feedback include vibrations occurring at a tip of the instrument (102) identified by one or more of discontinuity information or frequency response information in a twist profile representing the amount of twist along the instrument, said twist profile being derived from the optical shape sensing signal.

3. The system as recited in claim 1 or 2, further comprising a composite metric (524) configured to determine interaction between the instrument and the surface using two or more of said modes of the shape sensing feedback.

4. The system as recited in claim 1, further comprising a representation (134) of the surface configured for comparison with positions and orientations determined by the optical shape sensing of the instrument to determine whether surface contact has occurred or to identify a position where surface contact has occurred between the instrument and the surface.

5. The system as recited in claim 1, further comprising an actuation module (140) configured to adjust the instrument in accordance with the modes of the shape sensing feedback that identify whether contact is made with the surface.

6. The system as recited in claim 1, further comprising:

   a processor (114);
   memory (116) coupled to the processor and storing the interaction evaluation module (148); and
   an actuation module (140) configured to adjust the instrument (102) in accordance with the modes of the shape sensing feedback.

7. A method for detecting instrument interaction with a surface, comprising:

   receiving (502) shape sensing feedback from a flexible shape sensing enabled instrument configured to pass along the surface and which includes an optical shape sensing system (104) configured to provide an optical shape sensing signal as the shape sensing feedback; and
   evaluating (504) the shape sensing feedback using an interaction evaluation module configured to monitor the shape sensing feedback from the instrument to determine modes of the shape sensing feedback in order to identify whether contact is made with the surface and to determine the position of the contact,
   **characterized in that** the modes of shape sensing feedback include one or more of:

   vibrations identified by one or more of discontinuity information or frequency response information in the optical shape sensing signal;
   a shape configuration determined from the optical shape sensing signal indicating contact between the instrument (102) and the surface; and
   a motion profile determined from the optical shape sensing signal indicating contact between the instrument (102) and the surface.

**Patentansprüche**

1. Ein System zum Erfassen der Interaktion eines Instruments mit einer Oberfläche, das Folgendes umfasst:

   ein Formerfassungsinstrument (102), das entlang einer Oberfläche geführt wird, und das ein optisches Formerfassungssystem (104) umfasst, mit dem ein optisches Formerfassungssignal als Formerfassungs-Feedback bereitgestellt wird; und
   ein Interaktionsauswertungsmodul (148), das das Formerfassungs-Feedback vom Instrument überwacht, um die Modi des Formerfassungs-Feedbacks zu ermitteln, anhand derer ermittelt wird, ob und an welcher Position ein Kontakt mit der Oberfläche hergestellt wird,
   das sich dadurch auszeichnet, dass die Modi des Formerfassungs-Feedbacks mindestens eines der folgenden Merkmale aufweisen:

   Schwingungen, die anhand mindestens einer **Diskontinuität**sinformation oder **Frequenzgang** information des optischen Formerfassungssignals ermittelt werden;
eine anhand des optischen Formerfassungssignals ermittelte **Formkonfiguration,** die einen **Kontakt** zwischen dem Instrument (102) und der Oberfläche angibt; und
ein anhand des optischen Formerfassungssignals ermitteltes **Bewegungsprofil,** das einen **Kontakt** zwischen dem Instrument (102) und der Oberfläche angibt.

2. Das System gemäß Anspruch 1, wobei die Modi des Formerfassungs-Feedbacks Schwingungen umfassen, die an einer Spitze des Instruments (102) auftreten, und die anhand mindestens einer Diskontinuitätsinformation oder Frequenzganginformation in einem Verdrehungsprofil identifiziert werden, das den Umfang der Verdrehung entlang des Instruments angibt, wobei das Verdrehungsprofil aus dem optischen Formerfassungssignal abgeleitet wird.

3. Das System gemäß Anspruch 1 oder 2, das zudem eine zusammengesetzte Metrik (524) umfasst, die die Wechselwirkung zwischen dem Instrument und der Oberfläche mithilfe von mindestens zwei der Modi des Formerfassungs-Feedbacks ermittelt.

4. Das System gemäß Anspruch 1, das zudem eine Darstellung (134) der Oberfläche umfasst, mit der die Positionen und Ausrichtungen verglichen werden können, die bei der optischen Formerfassung des Instruments ermittelt wurden, um zu ermitteln, ob ein Oberflächenkontakt stattgefunden hat, oder um eine Position zu identifizieren, an der ein Oberflächenkontakt zwischen dem Instrument und der Oberfläche stattgefunden hat.

5. Das System gemäß Anspruch 1, das zudem ein Betätigungsmodul (140) umfasst, das das Instrument gemäß den Modi des Formerfassungs-Feedbacks einstellt, anhand derer ermittelt wird, ob ein Kontakt mit der Oberfläche vorliegt.

6. Das System gemäß Anspruch 1, das zudem Folgendes umfasst:

   einen Prozessor (114);
   einen Speicher (116), der mit dem Prozessor verbunden ist, und auf dem das Interaktionsauswertungsmodul (148) gespeichert wird; und
   ein Betätigungsmodul (140), das das Instrument (102) gemäß den Modi des Formerfassungs-Feedbacks einstellt.

7. Eine Methode zum Erfassen der Interaktion eines Instruments mit einer Oberfläche, die folgende Schritte umfasst:

   Abrufen (502) eines Formerfassungs-Feedbacks von einem flexiblen Formerfassungsinstrument, das entlang einer Oberfläche geführt wird, und das ein optisches Formerfassungssystem (104) umfasst, mit dem ein optisches Formerfassungssignal als Formerfassungs-Feedback bereitgestellt wird; und
   Auswerten (504) des Formerfassungs-Feedbacks mit einem Interaktionsauswertungsmodul, das das Formerfassungs-Feedback vom Instrument überwacht, um die Modi des Formerfassungs-Feedbacks zu ermitteln, anhand derer ermittelt wird, ob und an welcher Position ein Kontakt mit der Oberfläche hergestellt wird,
   das sich dadurch auszeichnet, dass die Modi des Formerfassungs-Feedbacks mindestens eines der folgenden Merkmale aufweisen:

   Schwingungen, die anhand mindestens einer Diskontinuitätsinformation oder Frequenzganginformation des optischen Formerfassungssignals ermittelt werden;
   eine anhand des optischen Formerfassungssignals ermittelte Formkonfiguration, die einen Kontakt zwischen dem Instrument (102) und der Oberfläche angibt; und
   ein anhand des optischen Formerfassungssignals ermitteltes Bewegungsprofil, das einen Kontakt zwischen dem Instrument (102) und der Oberfläche angibt.

**Revendications**

1. Système de détection de l'interaction d'un instrument avec une surface, comprenant :

   un instrument (102) activé par détection de forme conçu pour passer le long de la surface et lequel comprend un système (104) de détection de forme optique conçu pour fournir un signal de détection de forme optique en tant que rétroaction de détection de forme ; et

un module d'évaluation d'interaction (148) conçu pour surveiller le retour de détection de forme de l'instrument pour déterminer les modes du retour de détection de forme pour identifier si un contact est établi avec la surface et pour déterminer la position du contact,
**caractérisé en ce que** les modes de rétroaction de détection de forme comprennent :

des vibrations identifiées par des informations de **discontinuité** et/ou des informations de réponse en **fréquence** dans le signal de détection de forme optique ; et/ou
une **configuration de forme** déterminée à partir du signal de détection de forme optique indiquant le **contact** entre l'instrument (102) et la surface ; et/ou un **profil de mouvement** déterminé à partir du signal de détection de forme optique indiquant le contact entre l'instrument (102) et la surface.

2. Système selon la revendication 1, dans lequel les modes de rétroaction de détection de forme comprennent des vibrations se produisant à une pointe de l'instrument (102) identifiées par des informations de discontinuité et/ou des informations de réponse en fréquence dans un profil de torsion représentant la quantité de torsion le long de l'instrument, ledit profil de torsion étant dérivé du signal optique de détection de forme.

3. Système selon la revendication 1 ou 2, comprenant en outre une métrique (524) composite conçue pour déterminer l'interaction entre l'instrument et la surface à l'aide des au moins deux desdits modes de rétroaction de détection de forme.

4. Système selon la revendication 1, comprenant en outre une représentation (134) de la surface conçue pour être comparée avec des positions et des orientations déterminées par la détection optique de forme de l'instrument pour déterminer si un contact de surface s'est produit ou pour identifier une position où le contact de surface s'est produit entre l'instrument et la surface.

5. Système selon la revendication 1, comprenant en outre un module d'actionnement (140) conçu pour ajuster l'instrument selon les modes de rétroaction de détection de forme, lesquels identifient si un contact est établi avec la surface.

6. Système selon la revendication 1, comprenant en outre :

un processeur (114) ;
une mémoire (116) couplée au processeur et stockant le module d'évaluation d'interaction (148) ; et
un module d'actionnement (140) conçu pour ajuster l'instrument (102) en fonction des modes de rétroaction de détection de forme.

7. Procédé de détection de l'interaction d'un instrument avec une surface, comprenant :

la réception (502) d'une rétroaction de détection de forme d'un instrument flexible activé par détection de forme conçu pour passer le long de la surface et lequel comprend un système (104) de détection de forme optique conçu pour fournir un signal de détection de forme optique en tant que rétroaction de détection de forme ; et
l'évaluation (504) de la rétroaction de détection de forme à l'aide d'un module d'évaluation d'interaction conçu pour surveiller la rétroaction de détection de forme provenant de l'instrument pour déterminer les modes de rétroaction de détection de forme pour identifier si un contact est établi avec la surface et pour déterminer la position du contact,
**caractérisé en ce que** les modes de rétroaction de détection de forme comprennent :

des vibrations identifiées par des informations de discontinuité et/ou des informations de réponse en fréquence dans le signal de détection de forme optique ; et/ou
une configuration de forme déterminée à partir du signal de détection de forme optique indiquant le contact entre l'instrument (102) et la surface ; et/ou un profil de mouvement déterminé à partir du signal de détection de forme optique indiquant le contact entre l'instrument (102) et la surface.

Workstation - 112

Memory - 116

Optical sensing and interpretation module 115

Interaction evaluation module 148

Models, metrics, thresholds, etc. 136

Image 134

Database 138

Processor 114

Display 118

Interface 120

Optical interrogation 108

Optical source 106

127

100

Subject - 160

Volume - 131

Device - 102

Shape sensing - 104

Fiber - 126

Actuation module 140

Imaging system - 110

FIG. 1

FIG. 2

FIG. 3

FIG. 4

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8622935 B
- WO 2007015139 A